# EUROPEAN PATENT APPLICATION

(11) **EP 2 248 497 A1**
(43) Date of publication of application: **10.11.2010**
(21) Application number: 09718136.6
(22) Date of filing: 25.02.2009
(51) Int. Cl.: A61F 13/15, A61F 13/472, A61F 13/511, A61F 13/539

(54) **ABSORBENT ARTICLE**

(30) Priority: 04.03.2008 JP 2008053666
(71) Applicant: Unicharm Corporation, Ehime 799-0111 (JP)
(72) Inventor: NISHIKAWA, Kumiko, Kanonji-shi Kagawa 769-1602 (JP); NODA, Yuki, Kanonji-shi Kagawa 769-1602 (JP); KURODA, Kenichiro, Kanonji-shi Kagawa 769-1602 (JP)
(74) Representative: Peter, Julian
(86) International application number: PCT/JP2009/053997
(87) International publication number: WO 2009/110483

(57) **Abstract**

To apply a colored recess part to an absorbent article such as sanitary napkin without worsening the soft and comfortable touch to skin and allowing the colorant from coming into direct contact with the skin of a wearer.

An absorbent article comprising a liquid-permeable sheet, a liquid-impermeable sheet, and an absorber sandwiched between the liquid-permeable sheet and the liquid-impermeable sheet, wherein a recess part is provided in the liquid-permeable sheet, at least a portion provided with the recess part has a colored layer in contact with the back surface of the liquid-permeable sheet, and the recess part appears in a color different from the portion other than the recess part. The recess part is provided by stacking the liquid-permeable sheet and the colored layer and applying embossing from the liquid-permeable sheet side to the region where the colored layer is present.

## Description

### TECHNICAL FIELD

The present invention relates to an absorbent article. More specifically, the present invention relates to an absorbent article, such as a sanitary napkin and diaper.

### BACKGROUND ART

An absorbent article having a side sheet subjected to design embossing is known (see Japanese Unexamined Patent Publication No. 2006-110225). The invention disclosed in Japanese Unexamined Patent Publication No. 2006-110225 is intended to efficiently produce an absorbent article without causing breakage of a sheet material, in which wrinkling or twisting does not readily occur, flexibility decreases less, touch to skin is good, stuffiness does not readily occur, an embossed part with high visibility is provided, and satisfactory leakage prevention is achieved. However, the embossed part disclosed in Japanese Unexamined Patent Publication No. 2006-110225 has the same color as that of the non-embossed part and visibility is not necessarily high.

An absorbent article having a side sheet in which a pattern is formed by printing is also known (see Japanese Unexamined Patent Publication No. 2006-181192). The invention disclosed in Japanese Unexamined Patent Publication No. 2006-181192 is intended to let a wearer know the presence of the side sheet by printing a pattern and providing a sense of security in terms of leakage prevention, and for keeping the printing ink from substantially contacting the skin of the wearer, a method of forming a recess part by an emboss roll and at the same time, transferring ink to the bottom of the recess part, a method of printing a pattern on the surface not in direct contact with the skin of the wearer, i.e., on the back surface of the side sheet, and a method of printing a pattern on the back surface sheet are disclosed.

### DISCLOSURE OF INVENTION

According to the technique disclosed in Japanese Unexamined Patent Publication No. 2006-110225, even if design embossing could be applied in a uniform state, the part subjected to embossing is white and in turn, the embossed part is also white or nearly white, although the embossed part differs in light transmittance from the non-embossing part. Accordingly, when the wearer changes the absorbent article, the design cannot be easily seen in a dark place without light, such as inside of a toilet room. Also, the method which may be considered for clearly showing the embossing has a problem that this can be coped with only by production conditions such as heat, pressure and clearance, as a result, the embossed part becomes hard and gives an uncomfortable feeling when touching the skin, such as prickling and rubbing.

In the case of the method disclosed in Japanese Unexamined Patent Publication No. 2006-181192 where a recess part is formed by an emboss roll and at the same time, ink is transferred to the bottom of the recess part, the process is complicated and not practical, for example, there may be a possibility that ink bleeding may occurs when forming the recess part and the ink attaches to the receiving roll and is transferred to a portion other than the recess part. The recess part may not be stably formed or the ink disposed in the recess part may transfer to the skin surface due to body pressure of the wearer. Furthermore, in the case of the method of printing a pattern on a surface not in direct contact with the skin of the wearer or printing a pattern on the back surface sheet, the pattern is visually recognized through the side sheet and this gives rise to a problem that the visibility of the pattern is not necessarily high.

On wearing a sanitary napkin, a young person not used to wearing one or a women obliged to replace the sanitary napkin in a short period of time often suffers from wearing it in an in correct position, such as sliding back and forth or askew, due to lack of experience or impatience, which leads to leakage or uncomfortable fitting. An emboss applied to the absorbing surface is provided in many cases for the effect of leakage prevention or the like, but in the case where the emboss depth is not stabilized, and for example, a rise is generated, this causes a problem that a sufficient sense of security cannot be obtained visually or the feeling of the article is not good.

The absorbent article of the present invention is an absorbent article comprising a liquid-permeable sheet, a liquid-impermeable sheet, and an absorber sandwiched between the liquid-permeable sheet and the liquid-impermeable sheet, wherein a recess part is provided in the liquid-permeable sheet, at least a portion provided with the recess part has a colored layer in contact with the back surface of the liquid-permeable sheet, and the recess part appears in a color different from the portion other than the recess part.

In a preferred embodiment of the present invention, the recess part is provided by simultaneously embossing the liquid-permeable sheet, the colored layer and the absorber; the recess part is provided by simultaneously embossing the liquid-permeable sheet and the colored layer; the absorbent article further has a second liquid-permeable sheet between the liquid-permeable sheet and the absorber and the recess part is provided by simultaneously embossing the liquid-permeable sheet, the colored layer and the second liquid-permeable sheet; the recess part is forming a compressed groove; the light transmittance of the liquid-permeable sheet is 70% or less; the thickness of the liquid-permeable sheet is from 0.2 to 1.5 mm under a load of 3 g/cm²; the colored layer comprises a hot-melt resin containing a colorant; and the colored layer contains from 0.1 to 10 parts by weight of a colorant based on 100 parts by weight of the hot-melt resin.

The method of the present invention is a method for producing an absorbent article which comprises a liquid-permeable sheet, a liquid-impermeable sheet, an absorber sandwiched between the liquid-permeable sheet and the liquid-impermeable sheet, and a colored layer provided on the liquid-permeable sheet surface on the absorber side, and is provided with a recess part appearing in a color different from the portion other than the recess part, the method comprising stacking a liquid-permeable sheet and a colored layer and applying embossing from the liquid-permeable sheet side to the region where the colored layer is present, thereby providing a recess part in the liquid-permeable sheet.

The absorbent article of the present invention has a recess part differing in the color and therefore, a pattern by the recess part is clearly visible. At the same time, a colored layer is disposed below a liquid-permeable sheet and this enables keeping the colored layer from coming into direct contact with the skin surface.

Embossing is applied to the liquid-permeable sheets and the colored layer which differ in color, and the recess part formed by the embossing causes the liquid-permeable sheets and the colored layer to come close and be press-bonded, so that in the recess part, the color of the colored layer can be easily recognized when viewed from the liquid-permeable sheet side.

### BRIEF DESCRIPTION OF DRAWINGS

Fig. 1 is a plan view of one embodiment of the absorbent article of the present invention.
Fig. 2 is a cross-sectional view of one embodiment of the absorbent article of the present invention.
Fig. 3 is a plan view of another embodiment of the absorbent article of the present invention.
Fig. 4 is a cross-sectional view of another embodiment of the absorbent article of the present invention.
Fig. 5 is a cross-sectional view of another embodiment of the absorbent article of the present invention.
Fig. 6 is a plan view of another embodiment of the absorbent article of the present invention.
Fig. 7 is a plan view of another embodiment of the absorbent article of the present invention.
Fig. 8 is a cross-sectional view of another embodiment of the absorbent article of the present invention.

### BEST MODE FOR CARRYING OUT THE INVENTION

The present invention is described below by referring to the drawings, but the present invention is not limited to those illustrated in the drawings.

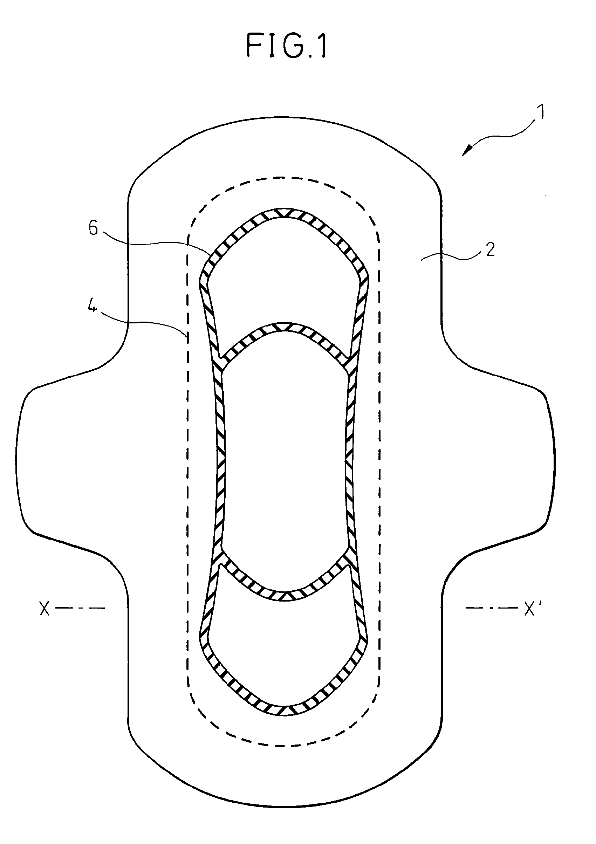

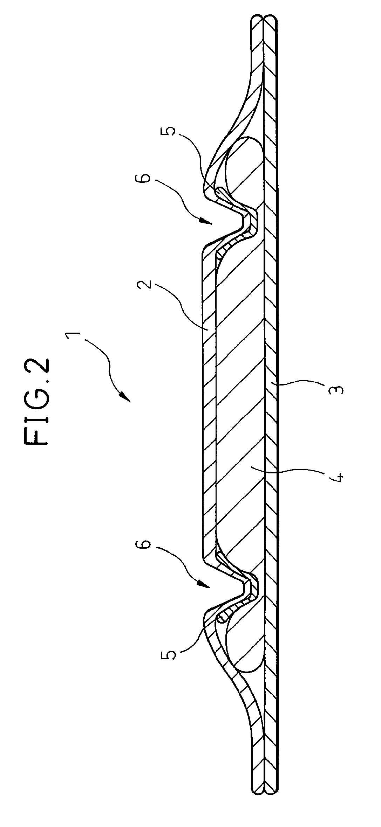

Fig. 1 is a plan view of one embodiment of the absorbent article of the present invention, and Fig. 2 is its X-X' cross-sectional view.

The absorbent article 1 of the present invention consists of a liquid-permeable sheet 2, a liquid-impermeable sheet 3 and an absorber 4. The absorber 4 is sandwiched between the liquid-permeable sheet and the liquid-impermeable sheet. In the liquid-permeable sheet 2, a recess part 6 is provided. At least in the portion where the recess part is provided, a colored layer 5 is provided in contact with the back surface of the liquid-permeable sheet 2. In the recess part 6, the liquid-permeable sheet 2 and the colored layer 5 are press-bonded and therefore, the recess part and the portion other than the recess part appear in different colors.

Fig. 1 illustrates an example of a sanitary napkin where a compressed groove is formed by a recess part. The compressed groove prevents transverse leakage of menstrual blood and promotes absorption of menstrual blood. The compressed groove may surround the middle or may be curved in the portion corresponding to the leg opening. Regarding such a compressed groove, for example, the wearer wears the napkin by attaching the surrounded portion in the middle expected to exert greatest absorption capacity to the area of excretion or wears the napkin by comforting the curve in the side part near the core to the curve of the crotch of shorts. At this time, when the compressed groove is colored and thereby highlighted, an inexperienced person or a person in a hurry can momentarily judge the absorbing surface even in a dark place such as the inside of a rest room and can correctly wear the napkin at the aimed position, so that leakage or an uncomfortable feeling due to slippage of the wearing position can be prevented. By coloring the compressed part, the surrounding configuration is highlighted more and the visibility of the compressed groove, which has been relied on for emboss strength, is augmented. As a result, a visual effect of alleviating the anxiety of leakage is obtained.

Such a colored compressed groove can be formed by simultaneously embossing the liquid-permeable sheet, the colored layer and the absorber to provide a recess part.

In the absorbent article 1 of the present invention, a second liquid-permeable sheet or a cushion layer may be provided between the liquid-permeable sheet 2 and the absorber 4.

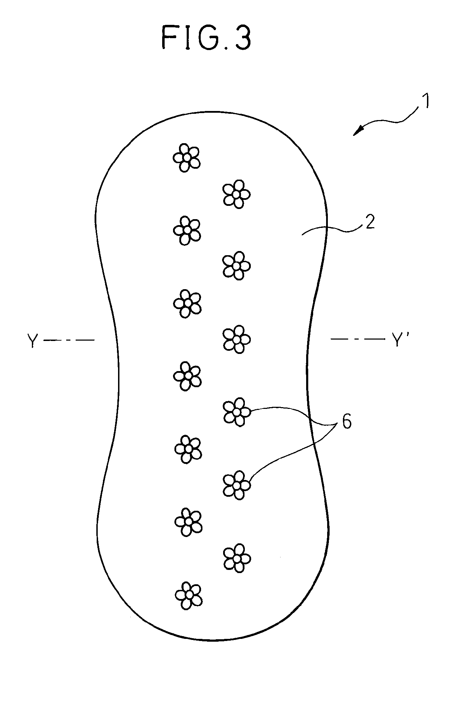

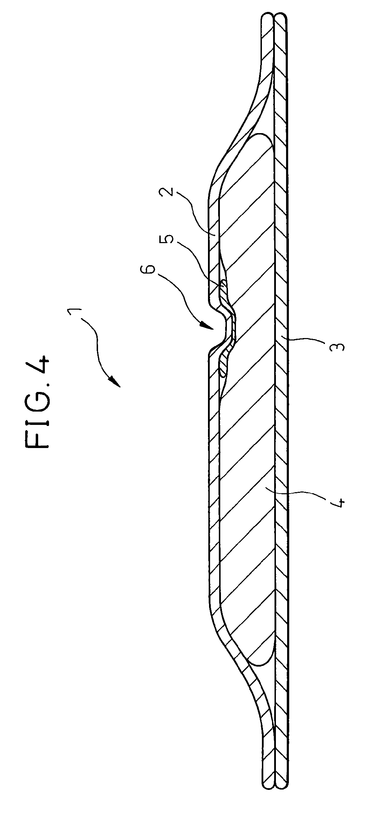

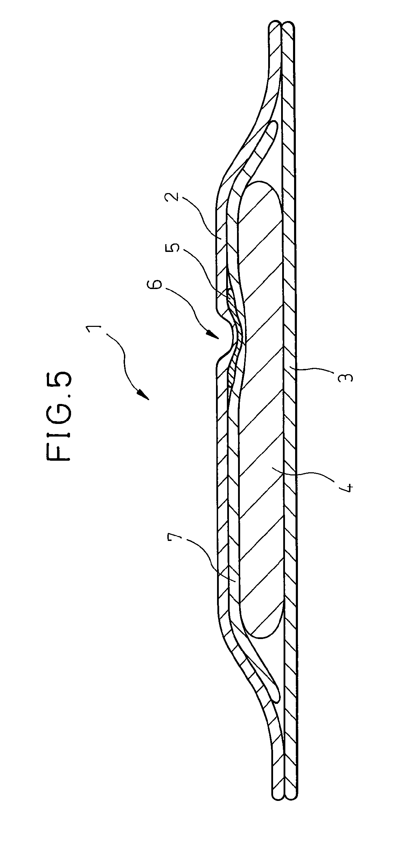

Fig. 3 illustrates an example of a sanitary napkin where a pattern is formed by a recess part on the liquid-permeable sheet surface. Figs. 4 and 5 each is its Y-Y' cross-sectional view. As illustrated in Fig. 3, recess parts 6 can be provided in the center part on the absorbing surface of the liquid-permeable sheet 2. Such recess parts can be provided by, as shown in Fig. 4, simultaneously embossing the liquid-permeable sheet 2 and the colored layer 5 or when as illustrated in Fig. 5, a second liquid-permeable sheet 7 is present, by simultaneously embossing the liquid-permeable sheet 2, the colored layer 5 and the second liquid-permeable sheet 7. In the case of applying embossing to the center part on the absorbing surface of the liquid-permeable sheet, the embossed part in particular is a region that is highly likely to contact with labia and clitoris, and the portion hardened by embossing is liable to contact a sensitive site and give a foreign-body feeling. In the present invention, by having a colored layer, even when embossing is performed without applying excessive heat or pressure, visibility of the recess part is easily obtained. Furthermore, a colored layer is provided in the menstrual blood absorbing region, so that not only a person not used to fitting of a napkin to shorts can momentarily recognize the absorbing region and fit the napkin to the correct position, but also discomfort during usage can be reduced by the effect of hiding the menstrual blood by the pattern. In addition to the pattern, such as the floral design illustrated in Fig. 3, a different configuration such as quilting-like or dot-like may be considered.

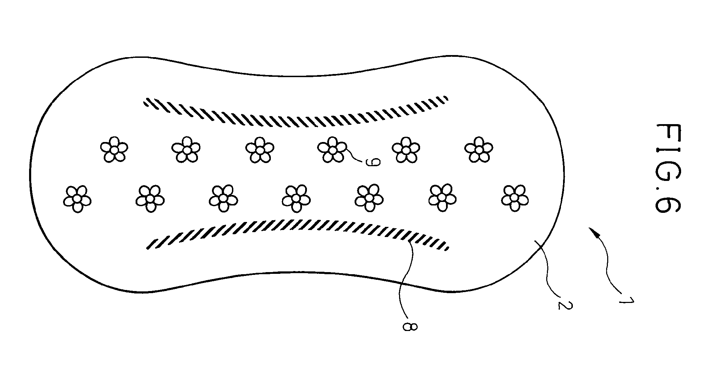

As illustrated in Fig. 6, both a compressed groove 8 and a pattern 9 in the center part on the absorbing surface may be provided. In this example, a liquid-permeable sheet, a second liquid-permeable sheet and an absorber are stacked in this order, and a colored layer is provided between the liquid-permeable sheet and the second liquid-permeable sheet. The napkin of this example can be produced by simultaneously embossing the liquid-permeable sheet, the colored layer and the second liquid-permeable sheet to form a recess part showing a colored pattern in the center part on the use-surface and then simultaneously embossing the liquid-permeable sheet, the colored layer, the second liquid-permeable sheet and the absorber to form a colored compressed groove.

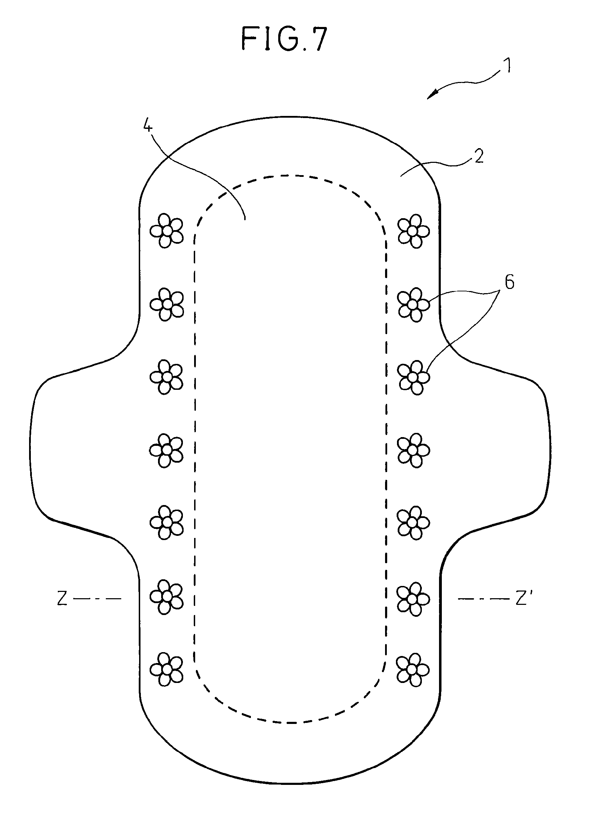

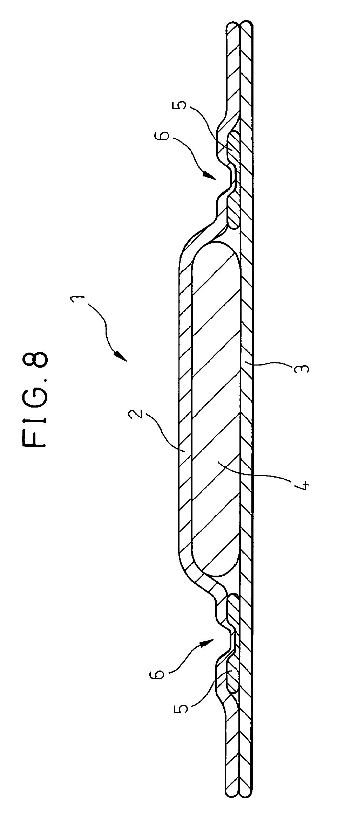

Fig. 7 illustrates an example of providing recess parts in a place where an absorber is not present. Fig. 8 is its Z-Z' cross-sectional view. Such recess parts 6 can be provided by simultaneously embossing the liquid-permeable sheet 2, the colored layer 5 and the liquid-impermeable sheet 3. In the case where a second liquid-permeable sheet is present between the colored layer and the liquid-impermeable sheet, the recess part can be provided by simultaneously embossing the liquid-permeable sheet, the colored layer and the second liquid-permeable sheet or by simultaneously embossing the liquid-permeable sheet, the colored layer, the second liquid-permeable sheet and the liquid-impermeable sheet.

The absorbent article of the present invention is preferably used as a sanitary napkin, a diaper or the like. In use as a sanitary napkin or a diaper, the absorbent article is worn such that the liquid-permeable sheet surface comes into contact with the skin of the wearer.

The absorbent article may have, for example, in the case of a sanitary napkin, a rectangular shape, an oval shape, a gourd shape, or a shape equipped with so-called wings for preventing slippage from the shorts and is not particularly limited in its shape as long as it fits to the women's body or the shape of shorts. The total outside dimension is preferably from 100 to 500 mm, more preferably from 150 to 350 mm, in the long direction and is preferably from 30 to 200 mm, more preferably from 40 to 180 mm, in the short direction.

The liquid-permeable sheet constituting the absorbent article of the present invention is used to exert a function of passing a liquid excretion from the body, such as menstrual blood and urine, down to the absorber provided as the underlying layer and at the same time, hold an absorber by sandwiching it between the liquid-permeable sheet and the liquid-impermeable sheet. The liquid-permeable sheet is entirely or partially liquid-permeable, and the liquid permeation area is formed of, for example, a resin film having formed therein a large number of openings for permeating liquid, a net-like sheet having a large number of meshes, or a liquid-permeable nonwoven or woven fabric. As for the resin film or net-like sheet, those formed from polypropylene (PP), polyethylene (PE), poly(ethylene terephthalate) (PET) or the like may be used. As to the nonwoven fabric, for example, a spunlaced nonwoven fabric formed from a cellulose fiber such as rayon, a synthetic resin fiber or the like, and an air-through nonwoven fabric formed from a synthetic resin fiber may be used.

A biodegradable natural product, such as poly(lactic acid), chitosan or poly(alginic acid) may be used as the material. Furthermore, in combination with forming a large number of openings for permeating liquid, a silicone-containing or fluorine-containing water-repellent oily agent may be coated to deter attachment of a body fluid to the outer surface.

The basis weight of the liquid-permeable sheet is preferably from 15 to 100 g/m², more preferably from 20 to 50 g/m², still more preferably from 25 to 40 g/m². If the basis weight is less than 15 g/m², sufficient surface strength may not be obtained and the sheet may rupture during usage, whereas if the basis weight exceeds 100 g/m², excessive roughness develops and an uncomfortable feeling occurs during usage. Furthermore, in use for a long time, if the basis weight exceeds 40 g/m², the liquid is held in the liquid-permeable sheet and stays in a sticky state and this leads to discomfort. The density is not particularly limited as long as it is 0.12 g/cm³ or less and the sheet is liquid-permeable. If the density exceeds this range, smooth permeation between fibers of the liquid-permeable sheet becomes difficult. In the case of menstrual blood, the viscosity is higher than urine or the like, and therefore the density is preferably low.

In the case where the liquid permeation area constituting the entirety or a part of the liquid-permeable sheet is a perforated film such as film having formed therein a large number of openings for permeating liquid, it is preferred that the opening diameter is from 0.05 to 3 mm, the pitch is from 0.2 to 10 mm, and the opening area percentage is from 3 to 30%.

If the light transmittance of the liquid-permeable sheet is too high, when a colored layer is present also in the portion not subjected to embossing, the color of the colored layer is seen through from the liquid-permeable sheet side and the recess part may not become distinguishable, despite appearing as a dark color. Accordingly, the light transmittance of the liquid-permeable sheet is preferably 70% or less, more preferably from 30 to 65%. The liquid-permeable sheet need not be white and may have a color other than white as long as the recess part can be visually recognized as different color-looking. The liquid-permeable sheet having a preferred light transmittance can be obtained, for example, by mixing a colorant in the liquid-permeable sheet. For example, in the case of decreasing the light transmittance of the white liquid-permeable sheet as it is, titanium oxide is used and the blending amount thereof is set to be from 0.1 to 50 % by weight, preferably from 1 to 10 % by weight, based on the entire fiber. The method for decreasing the light transmittance of the liquid-permeable sheet also includes a method of decreasing the diameter of the fiber constituting the nonwoven fabric. In this case, the appropriate size of the fiber is 6.6 dtex or less, preferably from 0.1 to 3.3 dtex.

As for the thickness (bulk) of the liquid-permeable sheet, when the liquid-permeable sheet is a nonwoven fabric, if the bulk is lost by embossing or the like, even a material having the same basis weight allows the color to be seen through the non-embossed part. The liquid-permeable sheet when compressed may be impregnated with a hot melt resin, and therefore the material used as the liquid-permeable sheet needs to have a certain degree of thickness/bulk. The thickness of the liquid-permeable sheet is preferably 0.2 mm or more, more preferably from 0.3 to 1.5 mm under a load of 3 g/cm².

A perforated nonwoven fabric material or a perforated film may also be used as the liquid-permeable sheet. In this case, three kinds of states are obtained, i.e., originally, the color of the colored layer is viewed directly from the openings and the color is seen through in the non-open parts, and when embossing is applied, the embossed part appears as a dark color. As a result, the open portions of the openings can also be looked like a pattern. The perforated sheet tends to be increased in bulk by the formation of a rib part, and as described above, this is effective in maintaining the liquid-permeable sheet at a distance from the colored layer. In the case of using a liquid-impermeable film mainly comprising polyethylene, polypropylene or the like, the light transmittance of the film itself is low, but the thickness is small. However, by adhering a rolled-up and wrinkled film to the colored layer or applying a gearing process so as to maintain the liquid-permeable sheet at a distance from the colored layer, seeing the color of the colored layer through the non-recess part can be reduced.

In the absorbent article of the present invention, a second liquid-permeable sheet may be further provided between the liquid-permeable sheet and the absorber. For the second liquid-permeable sheet, a sheet formed of a material similar to the above-described liquid-permeable sheet (for example, nonwoven fabric) and having a density slightly higher than that of the above-described liquid-permeable sheet is used, and this sheet may be provided so as to accelerate the movement of liquid to the absorber or prevent the liquid from being released from the absorber.

In the absorbent article of the present invention, a cushion layer may be further provided between the liquid-permeable sheet 2 and the absorber 4 or between the second liquid-permeable sheet 7 and the absorber 4.

The liquid-impermeable sheet constituting the absorbent article of the present invention has a function of preventing liquid such as menstrual blood and urine absorbed by the absorber from leaking outside, and a material capable of preventing outside leakage of such a liquid is used. When a material that does not pass a liquid, but having air permeability is used, stuffiness when wearing can be reduced and discomfort during wearing can be relieved. Examples of such a material include a liquid-impermeable film mainly comprising polyethylene (PE) or polypropylene (PP), an air-permeable film, and a composite sheet obtained by laminating a liquid-impermeable film on one surface of a nonwoven fabric such as spunbonded fabric. Preferably, a hydrophobic nonwoven fabric, a water-impermeable plastic film, a laminate sheet of nonwoven fabric with water-impermeable plastic film, or the like may be used. An SMS nonwoven fabric where a highly water-resistant melt-blown nonwoven fabric is sandwiched between high-strength spunbonded nonwoven fabrics may also be used.

The absorber constituting the absorbent article of the present invention has a function of absorbing and holding a liquid such as menstrual blood and urine, and a bulky material that does not readily lose shape and causes less chemical irritation, is preferred. Examples thereof include an absorber composed of a fluffed pulp or an air-laid nonwoven fabric and a super-absorbent polymer. Instead of a fluffed pulp, for example, a chemical pulp, a cellulose fiber and an artificial cellulose fiber such as rayon and acetate may be used. The absorber includes a mixture of a pulp having a basis weight of 500 g/m² and a polymer having a basis weight of 20 g/m² (the polymer is dispersed in the entirety), in which the pulp and the polymer are uniformly distributed over all and which is wrapped with a tissue having a basis weight of 15 g/m². Examples of the air-laid nonwoven fabric include a nonwoven fabric in which pulp and a synthetic fiber are thermally fused or bonded by a binder. The super-absorbent polymer (SAP) has a three-dimensional network structure in which a water-soluble polymer is appropriately crosslinked, and this polymer absorbs hundreds to thousands of times of water, but is substantially water-insoluble and does not release the once absorbed water even when pressure is applied. Examples thereof include starch-based, acrylic acid-based and amino acid-based particulate or fibrous polymers. The shape and structure of the absorber may be changed as needed, but the total liquid absorption amount of the absorber needs to cope with the designed insertion amount as an absorbent article and the desired usage. The size, absorption capacity and the like of the absorber are varied according to the usage.

The colored layer constituting the absorbent article of the present invention needs to be disposed at least in the portion where a recess part is provided, but the range in which the colored layer is disposed may be the whole or a part of the liquid-permeable sheet, or the colored layer may be disposed to create gradation.

The colored layer needs to be in a color different from that of the liquid-permeable sheet. This color is selected by taking into consideration the psychological effect but may be any color. The material constituting the colored layer is not particularly limited as long as it is a colored material such as an ink, a coloring material, a resin containing a colorant (a colored resin), a nonwoven fabric having kneaded therein a colorant or a nonwoven fabric having coated on the surface thereof a colorant (a colored nonwoven fabric), or a film having kneaded therein a colorant or a film having coated on the surface thereof a colorant (a colored film), but the material is preferably a hot-melt resin containing a colorant. A material obtained by forming a colorant-containing hot-melt resin into a film or sheet may also be used as the colored layer. In the case where a colorant-containing hot-melt resin is used as the colored layer, when heat embossing is applied from the liquid-permeable sheet, the hot-melt resin constituting the colored layer is melted due to heat applied during press-bonding and allowed to penetrate into and be fixed on the liquid-permeable sheet and therefore, the embossed part appears in a dark color as compared with its peripheral site. In the case where a colored nonwoven fabric or a colored film is used as the colored layer, unlike the case of using a colorant-containing hot-melt resin, it does not occur that a hot-melt resin is melted due to heat and allowed to penetrate into and be fixed on the liquid-permeable sheet, but when embossing is applied and the liquid-permeable sheet having a certain degree of thickness is thereby joined to the colored layer, the liquid-permeable sheet comes close to the colored layer in the recess part and is fixed, and therefore the color tone of the colored layer becomes easily visible when viewed from the liquid-permeable sheet side of the recess part, as compared with the non-recess part. In order to maintain the press-bonded state of the liquid-permeable sheet to the colored layer, a hot-melt resin may be coated therebetween. The colored nonwoven fabric can be obtained, for example, by previously kneading a colorant composed of a pigment or the like into a resin constituting a synthetic fiber, spinning a fiber from the resin to obtain a colored fiber, and processing the fiber into a nonwoven fabric. The colored film can be obtained, similarly, by previously kneading a colorant composed of a pigment or the like into a resin constituting a film and forming the resin into a sheet.

As for the colorant, a dye and a pigment both may be used. Examples of the dye include a direct dye typified by C.I. Direct Blue or the like, a reactive dye typified by C.I. Reactive Blue or the like, and an acid dye typified by Blue No. 1 or the like. As for the pigment, either an inorganic pigment or an organic pigment may be used, and examples of the organic pigment include Red No. 404.

In the case where the colored layer is a colorant-containing hot-melt resin, the content of the colorant is preferably 0.1 parts by weight or more, more preferably from 0.1 to 10 parts by weight, based on 100 parts by weight of the hot-melt resin. If the content of the colorant is too small, the color of the recess part is light and the pattern becomes unclear, whereas if the content of the colorant is excessively large, the color in the portion other than the recess part also becomes dark and the pattern becomes unclear.

In the case where the light transmittance of the liquid-permeable sheet is high, the color of the colored layer is seen through the portion other than the recess part. When the mixing ratio of the colorant in the colored layer is low, the color tone of the recess part is not so different from that in the periphery thereof and the original purpose cannot be achieved. The matter of importance is the difference in color tone (color difference) between the recess part and the non-recess part. The range of the color difference between the non-recess part and the recess part, in which a difference in the color tone can be perceived, is ΔE=6 or more, preferably from ΔE=6 to ΔE=10. Incidentally, as for the color difference, a color difference base of a combination of the liquid-permeable sheet and the colored layer is measured using a colorimeter (manufactured by Minolta Co., Ltd.) and after removing the liquid-permeable sheet, only the colored layer is measured, whereby the color difference value ΔE can be measured. Describing ΔE indicative of the color difference, the L*a*b* color system is a color system standardized by International Commission on Illumination (CIE) in 1976, which is widely used at present in various fields for indicating the color of a thing. In the L*a*b* color system, the brightness is represented by L*, and the chromaticity indicating hue and chroma is represented by a* and b*. The direction of color is shown, for example, a* is red direction, -a* is green direction, b* is yellow direction and -b* is blue direction. As the numerical value increases, the color becomes brighter. In the case of L*a*b* color system, the color difference can be indicated by the numerical value of ΔE*ab, and a color difference of two colors can be indicated by one numerical value. The calculation formula is ΔE*ab=[(ΔL*)2+(Δa*)2+(Δb*)2]^{1/2}.

With respect to the colored layer, when importance is attached to how it looks by a color difference, the liquid-permeable sheet may be white but need not be white. For example, also by using a color opposite the color of the colored layer in terms of hue for the liquid-permeable sheet, the above-described contrast can be made large. Furthermore, by using such different colors, these two colors are mixed in the recess part and a third color is formed. For example, when the liquid-permeable sheet is red and the colored layer is blue, the recess part is colored violet.

The absorbent article of the present invention can be produced by stacking a liquid-permeable sheet and a colored layer and applying embossing from the liquid-permeable sheet side to the region where the colored layer is present, to provide a recess part in the liquid-permeable sheet. In this case, after stacking a liquid-impermeable sheet, an absorber, a colored layer and a liquid-permeable sheet in this order, embossing may be applied from the liquid-permeable sheet side to the region where the colored layer is present; after stacking an absorber, a colored layer and a liquid-permeable sheet in this order, embossing may be applied from the liquid-permeable sheet side to the region where the colored layer is present and thereafter, the embossed absorber and liquid-permeable sheet may be stacked on a liquid-impermeable sheet; or after stacking a liquid-permeable sheet and a colored layer, embossing may be applied from the liquid-permeable sheet side to the region where the colored layer is present and thereafter, a liquid-impermeable sheet, an absorber and the embossed liquid-permeable sheet may be stacked in this order such that the colored layer comes between the absorber and the liquid-permeable sheet. In other words, the liquid-impermeable sheet and/or the absorber may be stacked on the colored layer and the liquid-permeable sheet before applying embossing or may be stacked on the colored layer and the liquid-permeable sheet after applying embossing.

The colored layer may be previously provided at least in a portion of the liquid-permeable sheet, where a recess part is intended to be provided. In this case, the liquid-permeable sheet previously provided with the colored layer is subjected alone or after stacking the absorber such that the liquid-permeable sheet surface having provided thereon the colored layer faces the absorber, is subjected to embossing of the region where the colored layer is present, from the liquid-permeable sheet side.

The colored layer may also be previously provided at least in a portion of the absorber, where a recess part is intended to be provided. In this case, the liquid-permeable sheet is stacked on the absorber surface having provided thereon the colored layer, and embossing is applied from the liquid-permeable sheet side to the region where the colored layer is present.

After preparing a two-layer sheet where the liquid-permeable sheet and the colored layer are previously stacked, the two-layer sheet may be subjected alone or after stacking the absorber such that the two-layer sheet surface on the colored layer side faces the absorber, may be subjected to embossing from the liquid-permeable sheet side.

Furthermore, after applying embossing to the liquid-permeable sheet to form a recess part, the colored layer may be disposed on the back surface of the liquid-permeable sheet, and the absorber and the liquid-impermeable sheet may be disposed therebelow. In this case, the portion of the recess part provided in the liquid-permeable sheet becomes thinner than the portion other than the recess part to make more see-through the colored layer disposed therebelow and therefore, the recess part appears in a color different from the portion other than the recess part.

Preferably, a colorant-containing hot-melt resin is used as the colored layer, the colored layer is previously coated at least on a portion of the liquid-permeable sheet where a recess part is intended to be provided, the liquid-permeable sheet coated with the colored layer is stacked on the absorber such that the liquid-permeable sheet surface on the side provided with the colored layer faces the absorber, and embossing is applied from the liquid-permeable sheet side to the region where the colored layer is present.

In the case of using a colorant-containing hot-metal resin, a new design can be created by the combination of the coating pattern of the hot-melt resin and the design embossing. Examples of the method for coating a colorant-containing hot-melt resin on the liquid-permeable sheet include solid pattern forming (no omission) by a slot coater, pattern forming with omission (striped) by a slot coater, control seam coating (wavy), spiral coating, and design coating by a roll coater.

An embossed pattern may be a solid pressed pattern or a fine line design. Embossing may contain a message to the wearer. How the pattern of the embossed part looks varies according to the coating method of the hot-melt resin. The coating pattern of the hot-melt resin is changed in the same emboss configuration, and the configuration of the coating pattern of the hot-melt resin is utilized as a design and combined with the design of embossing, whereby a new design can be visually recognized.

In the case of the solid coating pattern, the portion not coated with the hot-melt resin is not present and therefore, this coating pattern is suitable for both a solid pressed pattern and a design by a fine line. This coating pattern can also cope with a design extending in the longitudinal direction of the product. A mark for the prevention of mixing up of the front and back may be attached by using a design showing the front and back clearly, such as an arrow, or a letter can be highlighted.

In the case of the coating pattern by a slot coater, a design requiring press-bonding of a relatively large area at a time is suitable. Such a design includes a sold pressed pattern and a pattern where characters, pictures or the like are obliquely disposed, though the area becomes small. The pattern can, for example, give the user an image of preventing leakage by highlighting the side or making the absorption center portion look wide. In this coating method, the hardness of the laminated product is reduced due to the presence of a non-coated portion. A design extending in the longitudinal direction of the product is not suitable, because the non-coated part is also extending in the longitudinal direction and it is difficult to emboss the design on the coated part.

In the case of the control seam coating, the coated part is wavy and therefore, the same designs as in the coating pattern by a slot coater are suitable.

In the case of the spiral coating, the resin is evenly coated similarly to the solid coating, and therefore the same designs as in the solid coating pattern are suitable. This coating is advantageous in that the sheet does not become hard, though a non-coated part is present and the color appears light compared with the solid coating.

In the case of the design coating by a roll coater, the roll coater can transfer a hot-melt resin having a design made by the coater itself, and therefore it can give a design which may break the sheet if the design is made by embossing, such as a line shape extending in the longitudinal direction, or impart a three-dimensional appearance by combining a design by a roll coater with a design by embossing.

Regarding the method for providing a recess part, embossing is usually used, and heat embossing is preferable. Heat embossing is applied at the same time to the liquid-permeable sheet and the colored layer which differ in color tone from each other and in a recess part formed by embossing, the color of the colored layer becomes easily visible as compared with the non-recess part, because the distance between the liquid-permeable sheet and the colored layer is small when seen from the liquid-permeable sheet side. In the case where the colored layer is a colored hot-melt resin, the hot-melt resin is melted due to heat during embossing and allowed to penetrate into the liquid-permeable sheet and be fixed by pressure, which is preferable. In the case of using a colored nonwoven fabric or film, unlike the case of using a hot-melt resin, the hot-melt resin is not melted due to heat and not allowed to penetrate into and be fixed to the liquid-permeable sheet, but by simultaneously embossing the liquid-permeable sheet and the colored layer, the liquid-permeable sheet having a certain degree of thickness is joined to the colored layer and the distance between them becomes small, and therefore the color tone of the colored layer in the recess part becomes easily visible compared with the non-recess part. Compared with the case of creating a pattern only by embossing without using coloration, embossing (temperature, pressure, clearance) can be made mild and this enables to prevent the embossed part and its periphery from becoming excessively hard, as a result, prickling or an uncomfortable feeling due to rubbing during wearing can be reduced and good skin touch can be obtained.

The embossing can be carried out using a commonly employed apparatus. For example, embossing is applied using an emboss roll having provided thereon protrusion parts in a desired pattern. Therefore, a plurality of members constituting the absorbent article may be joined and integrated at the same time as a pattern is provided. For example, simultaneously providing a recess part in the liquid-permeable sheet, the liquid-permeable sheet and the absorber may be bonded or the liquid-permeable sheet and the liquid-impermeable sheet may be bounded to each other. In the case where a colorant-containing hot-melt resin is used as the colored layer, embossing is preferably heat embossing and by the heat embossing, the liquid-permeable sheet and the absorber can be adhered via the colored layer. In the case of using a liquid-permeable sheet coated entirely with a colorant-containing hot-melt resin or using a two-layer sheet where a liquid-permeable sheet and a colorant-containing hot-melt resin are stacked, by heat embossing, the liquid-permeable sheet and the absorber can be adhered via the colored layer and at the same time, the liquid-permeable sheet and the liquid-impermeable sheet can be adhered.

### EXAMPLES

### Example 1

The sanitary napkin illustrated in the plan view of Fig. 1 and the cross-sectional view of Fig. 2 was produced as follows.

A colored layer was coated in a portion of a liquid-permeable sheet, where a recess part was intended to be provided. For the colored layer, a material obtained by mixing 1 part by weight of Blue No. 404 as a colorant with 100 parts by weight of a hot-melt resin containing a styrene-butadiene-styrene block copolymer as the base polymer was used.

An absorber 4 and the liquid-permeable sheet 2 provided with a colored layer were stacked in the order illustrated in Fig. 2. Stacking was then carried out arranging the liquid-permeable sheet surface on the side provided with the colored layer 5 to face the absorber 4. Incidentally, a hot-melt resin (adhesive) was coated on a necessary portion between respective members.

The stack obtained by stacking the members as above was heat-embossed by using a heat embossing roll to provide a recess part in the portion where the colored layer was present. Thereafter, a liquid-impermeable sheet 3 was stacked, and the stack was integrated and then cut into the shape illustrated in Fig. 1 to obtain a sanitary napkin.

The obtained sanitary napkin had a compressed groove formed by a recess part, and the recess part was blue and the portion other than the recess part was white.

### Example 2

Liquid-permeable sheets formed from various materials were evaluated for the thickness, the color difference (difference in the degree to which the color is seen through when the colored layer was stacked) and the entire light transmittance.

The materials prepared were an air-through nonwoven fabric (AT), a spunbonded fabric (PPSB), a film (material: polyethylene, thickness: about 30 µm), and a perforated film (PFW) (material: low-density polyethylene, thickness: about 0.45 mm, opening ratio: about 30%), and as for the air-through non-woven fabric, 5 kinds of fabrics were prepared, i.e., a fabric obtained by recovering a fiber having a fiber diameter of 2.2 dtex and a basis weight of 27 with aging to increase the bulk (AT1), a fabric having a fiber diameter of 1.6 dtex and a basis weight of 27 (AT2), a fabric having a fiber diameter of 1.6 dtex and a basis weight of 30 (AT3), a fabric having a fiber diameter of 2.2 dtex and a basis weight of 25 (AT4), and a fabric having a fiber diameter of 2.2 dtex and a basis weight of 30 (AT5).

The thickness was measured under a load of 3 g/cm² by using a thickness gauge manufactured by Peacok K.K.

As for the color difference, each material was stacked on a hot-melt resin (HMA) colored blue and the color difference was measured from the material side by using a colorimeter manufactured by Minolta Co., Ltd. At this time, the color difference base was that of the hot-melt resin alone. As the color difference value is larger, the difference in color tone between the liquid-permeable sheet and the colored layer is larger, i.e., the recess part can be more easily recognized as a pattern.

The total light transmittance TT (%) was measured by using a turbidimeter NDH-300A manufactured by Nippon Denshoku Industries Co., Ltd. The total light transmittance is the amount of light passing through the material, that is expressed by a ratio, and as this value is larger, the material transmits more light, i.e., the color of the colored layer can be more easily recognized with an eye when viewed from the liquid-permeable sheet.

The measurement results are shown in Table 1.

In Table 1, the ΔE value indicates the following.

From 0 to 0.5: Difference in color tone is scarcely recognized.

From 0.5 to 1.5: Difference in color tone is slightly recognized.

From 1.5 to 3.0: Difference in color tone is fairly recognized.

From 3.0 to 6.0: Difference in color tone is conspicuously recognized.

From 6.0 to 12: Difference in color tone is large. 12 or more: Difference in color tone is very large.

**Table 1**

| Material | Basis Weight | Thickness mm | Color Difference ΔE | | Total Light Transmittance % |
|---|---|---|---|---|---|
| | | | HMA 5 gsm | HMA 10 gsm | |
| AT1 | 27 | 1.35 | 2.94 | 6.72 | 66.3 |
| AT2 | 27 | 0.35 | 3.84 | 7.14 | 67.2 |
| AT3 | 30 | 0.53 | 4.53 | 8.1 | 57.3 |
| AT4 | 25 | 0.4 | 4.02 | 7.75 | 62.1 |
| AT5 | 30 | 0.75 | 4.76 | 8.02 | 59.2 |
| PPSB | 25 | 0.15 | 1.26 | 2.76 | 85.8 |
| Film | 25 | 0.03 | 4.39 | 7.95 | 61.9 |
| PFW | 23.5 | 0.45 | 2.36 | 5.36 | 70.3 |

According to the results shown in Table 1, even with materials having the same basis weight, when the bulky air-through nonwoven fabric (AT4) and the spunbonded fabric (PPSB) reduced in the thickness by embossing are compared, in the case of laying a colored layer below the liquid-permeable sheet, the color difference greatly differs (the range where difference in color tone is fairly recognized) and the spunbonded fabric reduced in the thickness by embossing gives a low ΔE value and allows the color of the colored layer to be easily seen through. When air-through nonwoven fabrics having the same basis weight but differing in the thickness (AT3 and AT5) are compared, AT3 having a small thickness but having a small fiber diameter shows a tendency that the color of the colored layer becomes difficult to visually recognize.

The thickness in the level where the colored layer is not seen through the non-embossed part is, in the case of an air-through nonwoven fabric, preferably 0.2 mm or more, more preferably from 0.3 to 1.5 mm.

As for the total light transmittance, it is seen from the results of the color difference ΔE that PPSB having a total light transmittance of 86% showing transparency to color is not suitable as the liquid-permeable sheet. When 5 kinds of air-through nonwoven fabrics are compared, the total light transmittance is preferably 70% or less, more preferably 65% or less.

### Example 3

An embossed sample was prepared using a colorant-mixed hot-melt resin, and how the color in the recess part looked was confirmed. A hot-melt resin using a styrene-butadiene-styrene block copolymer as the base polymer was used as the hot-melt resin, Blue No. 404 was used as the colorant, 5 kinds of colored hot-melt resins differing in the mixing ratio of the colorant (parts by weight of the colorant based on 100 parts by weight of the hot-melt resin) were prepared, and samples varied in color by changing the basis weight at coating were further prepared. An air-through nonwoven fabric (PET/PE, 27 gsm, titanium oxide was blended in a ratio of 2% based on the weight of the fiber) was stacked on the sample, and the stack was embossed. The color in the embossed part and the color in the non-embossed part were compared, and the color of the embossed part and the degree of see-through vision of the non-embossed part were evaluated.
A: Distinct difference in color and little see-through.
B: Difference in color is not so distinct.
C: Difference in color is scarcely recognized (the embossed part does not become dark, or the non-embossed part is excessively dark).

**Table 2**

| Overall Evaluation of Contrast and See-Through Vision | | | | | | |
|---|---|---|---|---|---|---|
| | | Mixing Ratio of Colorant | | | | |
| | | (parts by weight) | | | | |
| | | 0.05 | 0.50 | 5 | 8 | 15 |
| Basis Weight at Coating | 3 gsm | C | B | B | A | C |
| | 5 gsm | C | B | A | A | C |
| | 10 gsm | C | A | A | B | C |
| | 25 gsm | C | A | - | - | - |

In the overall evaluation of the color difference (contrast) between the embossed part and the non-embossed part and the degree of see-through vision of color of the colored layer from the liquid-permeable sheet in the non-embossed part, when the mixing ratio is 5% or less, the difference in color is not recognized unless the coating is performed at a high basis weight of 10 gsm or more. However, coating at a high basis weight causes a problem that, for example, the hot-melt resin bleeds out when folding the material by a guide plate generally called a "sailor", and therefore accumulates on the guide plate or the hot-melt resin is melted due to heat when applying embossing, and thus bleeds out due to pressure, causing the material to twine around the emboss roll. Accordingly, the preferred range for the mixing ratio of the colorant, where coating can be performed at 10 gsm or less and the embossed part can be clearly recognized owing to the contrast of color tone, is 0.1% or more, preferably 0.1 to 10%.

### INDUSTRIAL APPLICABILITY

The absorbent article of the present invention can be used as a sanitary napkin, a diaper and the like. The absorbent article of the present invention can provide an aesthetic value by virtue of a colored recess part provided therein. In particular, in the case where the absorbent article is a sanitary napkin, an effect of alleviating depression during menstruation is produced. Furthermore, a colored pattern is provided in the menstrual blood absorbing region and therefore, the sanitary napkin can be stably fitted to the correct position.

### Description of Numerical References

- 1: Absorbent article
- 2: Liquid-permeable sheet
- 3: Liquid-impermeable sheet
- 4: Absorber
- 5: Colored layer
- 6: Recess part
- 7: Second liquid-permeable sheet
- 8: Compressed groove
- 9: Pattern

## Claims

1. An absorbent article comprising a liquid-permeable sheet, a liquid-impermeable sheet, and an absorber sandwiched between the liquid-permeable sheet and the liquid-impermeable sheet, wherein a recess part is provided in the liquid-permeable sheet, at least a portion provided with the recess part has a colored layer in contact with the back surface of the liquid-permeable sheet, and the recess part appears in a color different from the portion other than the recess part.

2. The absorbent article according to claim 1, wherein the recess part is provided by simultaneously embossing the liquid-permeable sheet, the colored layer and the absorber.

3. The absorbent article according to claim 1, wherein the recess part is provided by simultaneously embossing the liquid-permeable sheet and the colored layer.

4. The absorbent article according to claim 1, wherein the absorbent article further has a second liquid-permeable sheet between the liquid-permeable sheet and the absorber and the recess part is provided by simultaneously embossing the liquid-permeable sheet, the colored layer and the second liquid-permeable sheet.

5. The absorbent article according to claim 2, wherein the recess part is forming a compressed groove.

6. The absorbent article according to any one of claims 1 to 5, wherein the light transmittance of the liquid-permeable sheet is 70% or less.

7. The absorbent article according to any one of claims 1 to 6, wherein the thickness of the liquid-permeable sheet is from 0.2 to 1.5 mm under a load of 3 g/cm²_{.}

8. The absorbent article according to any one of claims 1 to 7, wherein the colored layer comprises a hot-melt resin containing a colorant.

9. The absorbent article according to claim 8, wherein the colored layer contains from 0.1 to 10 parts by weight of a colorant based on 100 parts by weight of the hot-melt resin.

10. A method for producing an absorbent article which comprises a liquid-permeable sheet, a liquid-impermeable sheet, an absorber sandwiched between the liquid-permeable sheet and the liquid-impermeable sheet, and a colored layer provided on the liquid-permeable sheet surface on the absorber side, and is provided with a recess part appearing in a color different from the portion other than the recess part, the method comprising stacking a liquid-permeable sheet and a colored layer and applying embossing from the liquid-permeable sheet side to the region where the colored layer is present, thereby providing a recess part in the liquid-permeable sheet.
